(19) Europäisches Patentamt
European Patent Office
Office européen des brevets

(11) Veröffentlichungsnummer: **0 547 523 B1**

(12) # EUROPÄISCHE PATENTSCHRIFT

(45) Veröffentlichungstag der Patentschrift: **17.05.95**

(51) Int. Cl.[6]: **C07D 405/04**, A61K 31/35, A61K 31/40, A61K 31/445

(21) Anmeldenummer: **92121155.3**

(22) Anmeldetag: **11.12.92**

(54) **Ethanoladdukte von 6-sulfonylsubstituierten 3-Hydroxychromanen und ihre Verwendung als Inhalationsmittel bei Krankheiten.**

(30) Priorität: **14.12.91 DE 4141350**

(43) Veröffentlichungstag der Anmeldung:
**23.06.93 Patentblatt 93/25**

(45) Bekanntmachung des Hinweises auf die Patenterteilung:
**17.05.95 Patentblatt 95/20**

(84) Benannte Vertragsstaaten:
**AT BE CH DE DK ES FR GB GR IE IT LI LU MC NL PT SE**

(56) Entgegenhaltungen:
**EP-A- 0 277 612**
**EP-A- 0 351 720**
**EP-A- 0 412 760**
**WO-A-86/03750**

(73) Patentinhaber: **HOECHST AKTIENGESELLSCHAFT**

**D-65926 Frankfurt (DE)**

(72) Erfinder: **Englert, Heinrich, Dr.**
**Stormstrasse 13**
**W-6238 Hofheim/Ts. (DE)**
Erfinder: **Mania, Dieter, Dr.**
**Goethestrasse 43**
**W-6240 Königstein/Ts. (DE)**
Erfinder: **Kibat, Paul-Gerhard, Dr.**
**Kamilleweg 24**
**W-6200 Wiesbaden (DE)**
Erfinder: **Gehring, Doris**
**Frankfurter Strasse 166**
**W-6233 Kelkheim/Ts. (DE)**
Erfinder: **Paulus, Erich, Dr.**
**Lindenweg 26**
**W-6239 Eppstein/Ts. (DE)**

EP 0 547 523 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Ethanoladdukte der Verbindungen der Formel I

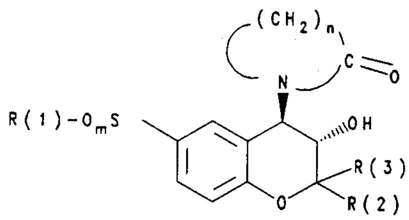

x $C_2H_5OH$

I

in denen

| | |
|---|---|
| R(1) | für Phenyl steht, das durch 1 oder 2 Methylgruppen und/oder Chlor substituiert sein kann, |
| R(2) und R(3) | gleich oder verschieden sind und für Methyl oder Ethyl stehen, sowie |
| n | für die Zahlen 3 und 4, |

sowie m für 1 und 2 steht.

Die Erfindung betrifft ausschließlich optisch aktive Verbindungen, bei denen der Lactamrest und die Hydroxygruppe die 4R- bzw. die 3S-Konfiguration aufweisen. Ist das C-2 Atom des Chromansystems asymmetrisch substituiert, so bezieht sich die Erfindung sowohl auf S- als auch auf R-konfigurierte Verbindungen.

Unter Ethanoladdukten versteht man stabile Solvate der Verbindungen I mit Ethanol in dem Sinne, daß Ethanol fester Bestandteil des Kristallgitters ist, durch die die jeweilige Verbindung in fester Form charakterisiert wird.

Besonders bevorzugt von den Verbindungen I ist das Ethanoladdukt von (3S,4R)-3-Hydroxy-2,2-dimethyl-4-(2-oxo-1-pyrrolidinyl)-6-phenylsulfonyl chroman.

Die Grundverbindungen der Formel I a

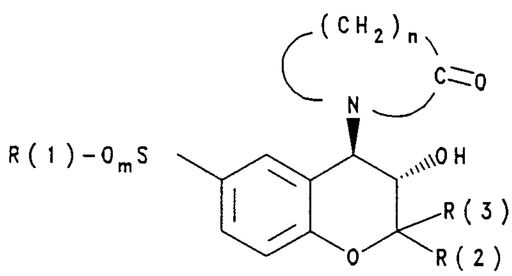

I a

sind bereits aus der Patentanmeldung EP 277 612 bekannt, und ihre Verwendung bei der Behandlung von Asthma ist in der Patentanmeldung EP 351 720 beschrieben. Es handelt sich dort jedoch nicht um Ethanoladdukte, sondern entweder um solvatfreie Verbindungen I oder um deren Hydrate. Bei den dort vorgeschlagenen Anwendungen der Verbindungen I a, wie etwa bei Asthma, bei der die Substanzen bevorzugt durch Inhalation verabreicht werden, ist dies jedoch auf eine Vernebelung von Lösungen der Substanzen beschränkt, beispielsweise von wässrigen Lösungen. Dies ist bei der sehr begrenzten Wasserlöslichkeit der Verbindungen I a jedoch nur beschränkt durchführbar. Wesentlich vorteilhafter wäre eine Inhalation der pulverförmigen Substanzen, entweder direkt oder als deren Suspension in einem Fluorchlorkohlenwasserstoff (FCKW) als Treibgas, die eine wesentlich höhere und raschere Dosierung erlauben würde. Um eine gute Lungengängigkeit von als Pulver inhalierten Substanzen zu gewährleisten, muß in solchen Fällen eine Mikronisierung durchgeführt werden. Hierbei hat es sich gezeigt, daß Substanzen nach EP 351 720 wegen ihrer inhärenten Klebrigkeit mit den gängigen Mahlverfahren nicht oder nur sehr unvollständig mikronisierbar sind.

Aus der EP 0 412 760 sind Solvate von (3S,4R)-3-Hydroxy-2,2-dimethyl-4-(2-oxo-1-piperidinyl)6-ethylchroman bekannt; 6-Phenylsulfonyl-Verbindungen sind darin nicht erwähnt oder aus dieser Entgegenhaltung nahegelegt, ebensowenig wie Ethanolate.

Die WO-A-8603750 beschreibt Beclomethasondipropionat, also ein Steroid, und auch dessen Solvate mit niederen Alkoholen; Ethanolate von Chromanen werden weder beschrieben noch nahegelegt.

Überraschenderweise gelang es nun, für die Verbindungen I a deren bislang unbekannte Ethanoladdukte I herzustellen. Sie erwiesen sich als stabil, sehr gut mikronisierbar, und sie verändern ihre Partikelgröße im Gegensatz zu den aus EP 351 720 bekannten Substanzen nicht, wenn sie in einem FCKW oder verwandten Treibmitteln suspendiert werden. Damit haben die erfindungsgemäßen Ethanoladdukte wertvolle physikalische Eigenschaften, die bei ihrer Anwendung als Pulverinhalate vorteilhaft ausgenutzt werden können.

Strukturell ähnliche Verbindungen und ihre Solvate, bevorzugt ihre Hydrate sowie deren Anwendung bei Asthma werden beispielsweise auch in der Patentschrift EP 176 689 erwähnt, ohne daß jedoch auf Besonderheiten eines Solvates oder gar eines Ethanoladduktes für die Herstellung eines Pulverinhalates hingewiesen wurde oder ein solches je isoliert und beschrieben worden wäre.

Die Herstellung der erfindungsgemäßen Ethanoladdukte gelingt dadurch, daß man eine solvatfreie Verbindung der Formel Ia oder ein beliebig anderes Solvat der Verbindungen Ia, beispielsweise das Hydrat, mit Ethanol in Kontakt bringt, vorzugsweise dadurch, daß man eine solche Verbindung aus Ethanol umkristallisiert.

Die Synthese der für eine solche Umwandlung benötigten Ausgangsmaterialien ist entweder in der Anmeldung EP 277 612 beschrieben, oder deren Herstellung wird, wie für Beispiel 1 aufgezeigt in analoger Weise durchgeführt.

Wie bereits erwähnt, eignen sich die Ethanoladdukte besonders für eine Inhalationsanwendung etwa bei obstruktiven Atemwegserkrankungen, wie Asthma. Die tägliche Dosis liegt dabei bei Asthma je nach der Schwere der Krankheit bei etwa 0.1 µg/kg Körpergewicht bis etwa 100 µg/kg, wobei ein Bereich von 1-10 µg/kg besonders bevorzugt ist. Für eine Inhalationsanwendung wird das Ethanolat als mikronisiertes Pulver zur Verfügung gestellt, wobei eine Teilchengröße von mindestens 10 µm, bevorzugt jedoch von 3-8 µm, vorteilhaft ist. Ein solches Pulver wird entweder als solches mit oder ohne Zusatz von Hilfsstoffen, wie etwa Laktose, inhaliert bzw. mit der Atemluft angesaugt, wobei man sich eines geeigneten Geräts bedient. Es kann aber aber auch in einer Treibgaszubereitung, beispielsweise in einer Dosieraerosoldruckflasche Verwendung finden, wobei Additive - wie oberflächenaktive Stoffe oder andere Zusätze - benötigt werden können.

Beispiel 1

(3S,4R)-3-Hydroxy-2,2-dimethyl-4-(2-oxo-1-pyrrolidinyl)-6-phenylsulfonylchroman Ethanolat-Addukt

(3S,4R)-3-Hydroxy-2,2-dimethyl-4-(2-oxo-1-pyrrolidinyl)-6-phenylsulfonylchroman hemihydrat wird in siedendem Ethanol gelöst und langsam auf Zimmertemperatur abgekühlt. Das Produkt wird für 8 h bei 80 °C getrocknet. M.p.118-120 °C; IR (KBr, $cm^{-1}$) 1639, 1480, 1302, 1152, 1080, 941, 732, 608, 577; Anal. ber. für $C_{21}H_{23}NO_5$ x 1 $C_2H_5OH$ : C, 61.7; H, 6.5; N, 3.1; gef.: C, 61.5; H, 6.3; N, 3.2.

Herstellung der Ausgangsmaterials (3S,4R)-3-Hydroxy-2,2-dimethyl-4- (2-oxo-1-pyrrolidinyl)-6-phenylsulfonylchroman hemihydrat

13.2 g (0.03 Mol) (3S,4R)-4-(4-Chlorbutyrylamino)-3-hydroxy-2,2-dimethyl-6-phenylsulfonylchroman werden in 100 ml THF gelöst und mit 4 g fein gemahlenem festen NaOH versetzt. Die Reaktionsmischung wird für eine Stunde bei Zimmertemperatur gerührt, und nach Zugabe von 25 ml $H_2O$ wird im Vakuum eingengt. Der Rückstand wird mit Eiswasser behandelt bis zur beginnenden Kristallisation. Umkristallisation aus reinem Methanol und nachfolgend aus Methanol/$H_2O$ (1:2) ergibt das Hemihydrat Addukt.
Ausbeute: 10.0 g,
m.p. 121-123 °C,
$^1$H-NMR (270 MHz, $CDCl_3$): 1.25 (s, $CH_3$), 1.51 (s, $CH_3$), 2.00-2.18 (m, C(4')$H_2$), 2.47-2.70 (m, C(3')$H_2$), 2.95-3.05 (m, C(5')$H_2$),3.18-3.30 (ps-dd, C(5')$H_2$), 3.13 (d, J = 5.6 Hz, O-H), 3.71 (dd, J = 10 Hz, J' = 5.6 Hz, C(3)$H_2$), 5.31 (d, J = 10 Hz, C(4)$H_2$), 6.91 (d, J = 8.4 Hz, C(6)$H_2$), 7.45-7.60, 7.73, 7.9 (m, 7H, Ar-H);
$[\alpha]_D^{20}$ = +41 ° (c = 1, Ethanol),
IR (KBr, $cm^{-1}$) 1663, 1478, 1320, 939, 602;
Anal. ber. für $C_{21}H_{23}NO_5$ x 0.5 $H_2O$: C, 61.4; H, 5.9; N, 3.4; gef.: C, 61.4; H, 5.7; N, 3.4.

Herstellung weiterer Vorstufen:

(3S,4R)-4-(4-Chlorbutyrylamino)-3-hydroxy-2,2-dimethyl-6-phenylsulfonylchroman

15.8 g (0.033 Mol) (3S,4R)-4-Amino-3-hydroxy-2,2-dimethyl-6-phenylsulfonylchroman (+)-Mandelat gibt man zu einer gerührten Mischung aus 3.26 g NaOH in 80 ml $H_2O$ und 80 ml $CH_2Cl_2$. Nach Abkühlung auf 5°C fügt man 6.84 g (0.0485 Mol) 4-Chlorbutyrylchlorid zu und rührt die Mischung für weitere 30 min bei 5-10°C. Nach Zugabe von 100 ml $CH_2Cl_2$ (um bereits ausgefallenes Produkt wieder in Lösung zu bringen) werden die organischen Phasen abgetrennt und 2 mal mit 2N NaOH und nachfolgend mit $H_2O$ gewaschen. Nach Abdampfen des Lösungsmittels wird der Rückstand mit heißem Diisopropylether behandelt.
Ausbeute: 13.6 g; m.p.:178-180;
$[\alpha]_D^{20}$ = -25° (c = 1, Methanol),
Anal. ber. für $C_{21}H_{24}ClNO_5S$: C, 57.6; H, 5.5; N, 3.2; gef.: C, 57.4; H, 5.5; N, 3.2.

(3S,4R)-4-Amino-3-hydroxy-2,2-dimethyl-6-phenylsulfonylchroman (+)-Mandelat und freie Base

198 g (0.59 Mol) des racemischen 4-Amino-3-hydroxy-2,2-dimethyl-6-phenylsulfonylchromans und 90.28 g (0.59 Mol) (+)- Mandelsäure werden in 4.5 l heißem (60°C) absoluten Ethanol gelöst. Man läßt innerhalb von 2 h auf Zimmertemperatur abkühlen. Falls dabei nicht spontane Kristallisation auftritt, impft man die Lösung mit 0.3 g des optisch reinen Endproduktes an. Danach beläßt man die Lösung für 48 h bei Zimmertemperatur und saugt die erhaltenen Kristalle ab. Das so erhaltene Material mit $[\alpha]_D^{20}$ = +83.6° (c = 1, DMF) wird erneut in 1 l Ethanol für 1 h am Rückfluß gekocht und ergibt nach Abkühlen einheitliches (+)-Mandelate.
Ausbeute: 81 g;
m.p.: 203-204°C;
$[\alpha]_D^{20}$ = +94° (c = 1, DMF);
Anal. ber. für $C_{25}H_{27}NO_7S$: C, 61.8; H, 5.6; N, 2.9; gefunden: C, 62.1; H, 5.7; N, 3.0. Die entsprechende freie Base wurde erhalten, indem man das Mandelat in einer gerührten Mischung aus 2N NaOH and $CH_2Cl_2$ suspendierte. $[\alpha]_D^{20}$ = +86° (c = 1, DMF).

4-Amino-3-hydroxy-2,2-dimethyl-6-phenylsulfonylchroman

50 g (0.158 Mol) 3.4-Epoxy-2,2-dimethyl-6-phenylsulfonylchroman, gelöst in 280 ml Ethanol, werden in einem Autoklaven bei 70°C und 7 bar $NH_3$-Druck 18 h lang geschüttelt. Nach Verdampfen des Lösungsmittels wird der Rückstand aus Isopropanol umkristallisiert. Ausbeute: 39.9 g; M.p.: 166-167°C.

3,4-Epoxy-2,2-dimethyl-6-phenylsulfonylchroman

Zu 100 ml absoluten DMSO gibt man 1.8 g (0.06 Mol) NaH als eine 80 %ige Suspension in Öl sowie tropfenweise 20 g 3-Bromo-4-hydroxy-2,2-dimethyl-6-phenylsulfonylchroman, gelöst in 80 ml DMSO, wobei die Reaktionstemperatur bei 25-28°C gehalten wird. Nach 2 stündigem Rühren bei Zimmertemperatur wird die Mischung in Eiswasser eingerührt und der entstandene Niederschlag abgesaugt. Durch Lösen des Rohprodukts in Essigsäureethylester und Behandlung mit Aktivkohle kann eine weitere Reinigung erzielt werden. Nach Abdampfen des Lösemittels wird der Rückstand bis zur beginnenden Kristallisation mit Diisopropylether behandelt. Ausbeute: 14.5 g; m.p.: 103-105°C.

3-Bromo-4-hydroxy-2,2-dimethyl-6-phenylsulfonylchroman

Zu 200 g 4-Nitrodiphenylsulfon (0.76 Mol) und 140 ml (1.45 Mol) 2-Methyl-3-butin-3-ol in 1.4 l DMSO gibt man 100 g feste NaOH-Plätzchen. Die Mischung wird bei Zimmertemperatur 5 h lang gerührt, wobei weitere 50 ml 2-Methyl-3-butin-3-ol nach 2h Rührzeit zugegeben werden. Danach wird die Mischung in 1.5 l Diisopropylether und 1 l $H_2O$ eingerührt. Die organische Phase wird abgetrennt und 3 mal mit $H_2O$ gewaschen. Nach Abdampfen des Lösemittels verbleibt ein rötliches Öl. Durch Chromatographie an Kieselgel kann man eine kleine Probe rein erhalten: 2-Methyl-3-(4-phenylsulfonyl)phenoxy-3-butin, m.p.: 58-60°C; Anal. ber. für $C_{17}H_{16}O_3S$: C, 68.0; H, 5.4; gef.: C, 67.5; H, 5.8. Die Hauptmenge dieses Produktes wird ohne weitere Reinigung in 800 ml 1.2-Dichlorobenzol gelöst und für 2 h am Rückfluß gekocht. Nach Abdestillieren des Lösungsmittels im Vakuum bei 100°C wird ein dunkles Öl erhalten, das ebenfalls durch Chromatographie an Kieselgel zu reinem 2,2-Dimethyl-6-phenylsulfonylchromen, m.p.: 95-96°C; Anal. ber.

für $C_{17}H_{16}O_3S$: C, 68.0; H, 5.4; gef.: C, 68.3; H, 5.3 aufgereinigt werden kann.
Das von dieser Verbindung erhaltene Rohprodukt wird in 600 ml DMSO und 10 ml $H_2O$ gelöst und bei 15 °C mit 100.2 g N-Bromsuccinimid, aufgeteilt in kleinen Portionen, versetzt. Die Mischung wurde für insgesamt 5 h gerührt und danach auf Eiswasser gegossen. Extraktion mit Methyl-tert.butylether ergibt rohes 3-Bromo-4-hydroxy-2,2-dimethyl-6-phenylsulfonylchroman, das man durch Rühren in Diisopropylether rein gewinnt. Ausbeute über 3 Stufen: 105.3 g; m.p.: 122-124 °C

Pharmakologische Daten:

a) Mikronisierung

890 g
(3S,4R)-3-Hydroxy-2,2-dimethyl-4-(2-oxo-1-pyrrolidinyl)-6-phenylsulfonylchroman Ethanol-Addukt werden in einer Luftstrahlmühle mikronisiert. Die Partikelgröße betrug <10 µm für 50% der Partikel. Mit (3S,4R)-3-Hydroxy-2,2-dimethyl-4-(2-oxo-1-pyrrolidinyl)-6-phenylsulfonylchroman oder (3S,4R)-3-Hydroxy-2,2-dimethyl-4-(2-oxo-1-pyrrolidinyl)-6-phenylsulfonylchroman-hemihydrat ließ sich unter diesen Bedingungen keine Mahlung durchführen, da die Substanzen die Führungsgänge der Mühle verklebten.

b) Stabilität in FCKW:

Das unter a) erhaltene mikronisierte Material von (3S,4R)-3-Hydroxy-2,2-dimethyl-4-(2-oxo-1-pyrrolidinyl)-6-phenylsulfonylchroman Ethanolat-Addukt veränderte seine Teilchengröße nicht wenn es in einem FCKW für 8 Tage suspendiert wurde.

c) Bronchodilatierende Wirkung am narkotisierten Meerschweinchen

Das unter a) erhaltene mikronisierte Material von (3S,4R)-3-Hydroxy-2,2-dimethyl-4-(2-oxo-1-pyrrolidinyl)-6-phenylsulfonylchroman Ethanolat-Addukt wird in FCKW suspendiert und in ein Druckaerosol-Dosiergefäß eingeschlossen. Die Konzentration wird so gewählt, daß pro Sprühstoß eine Substanzmenge von 0.1mg bzw. 1.0 mg mit etwa 0.5 ml FCKW versprüht wird. Wird ein solcher Sprühstoß in den Einatemschlauch der Atempumpe der nach Konzett-Rössler Methode präparierten, mit Pentobarbital narkotisierten Meerschweinchen eingebracht, so kommt es zu einer 30 Minuten (n = 2) bzw 40 Min (n = 3) anhaltenden Reduktion der durch Histamin i.v. ausgelösten Bronchokonstriktion, wobei initial eine vollständige Hemmung beobachtet wurde. Die Details der Methode wurden wie in EP 351 720 beschrieben gestaltet.

## Patentansprüche

**1.** Ethanol-Addukte der Formel I

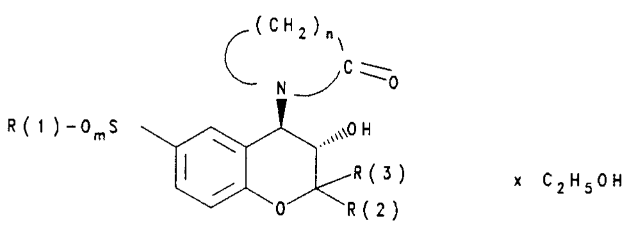

I

in denen

| | |
|---|---|
| R(1) | für Phenyl steht, das durch 1 oder 2 Methylgruppen und oder Chlor substituiert sein kann, |
| R(2) und R(3) | gleich oder verschieden sind und für Methyl oder Ethyl stehen, sowie |
| n | für die Zahlen 3 und 4, |

sowie m für 1 und 2 steht.

**2.** Verfahren zum Herstellen eines Ethanol-Adduktes I nach Anspruch 1, dadurch gekennzeichnet, daß man eine Verbindung I a

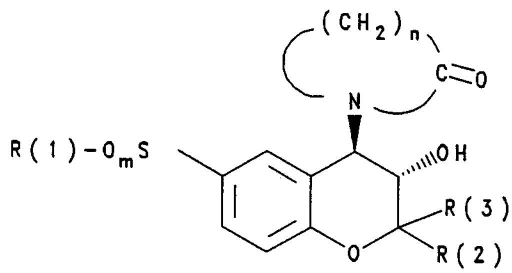

I a

oder eines ihrer Solvate mit Ethanol in Kontakt bringt.

**3.** Ethanol-Addukt der Formel I nach Anspruch 1

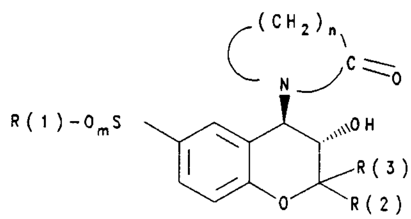

x $C_2H_5OH$

I

zur Verwendung als Inhalationsmittel zur Behandlung von Asthma.

**4.** Heilmittel, bestehend aus einer wirksamen Menge eines Ethanolats I nach Anspruch 1

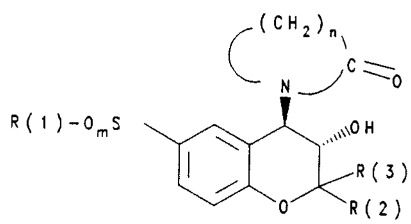

x $C_2H_5OH$

I

und pharmazeutisch üblichen Zuschlagmitteln.

## Claims

**1.** An ethanol adduct of the formula I

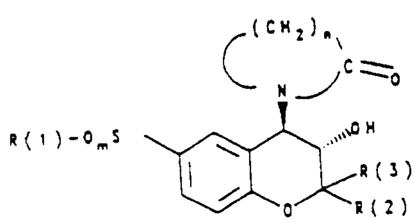

x $C_2H_5OH$

I

in which

| | |
|---|---|
| R(1) | is phenyl which can be substituted by 1 or 2 methyl groups and/or chlorine, |
| R(2) and R(3) | are identical or different and are methyl or ethyl, and |
| n | is the number 3 or 4, |

and m is 1 or 2.

**2.** A process for preparing an ethanol adduct I as claimed in claim 1, which comprises bringing a compound Ia

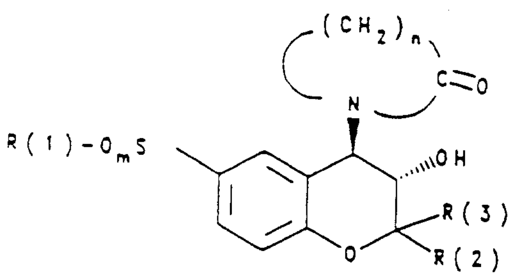

Ia

or one of its solvates into contact with ethanol.

**3.** An ethanol adduct of the formula I as claimed in claim 1

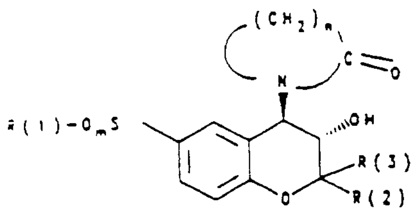

x $C_2H_5OH$

I

for use as an inhalant for the treatment of asthma.

**4.** A medicament composed of an effective amount of an ethanolate I as claimed in claim 1

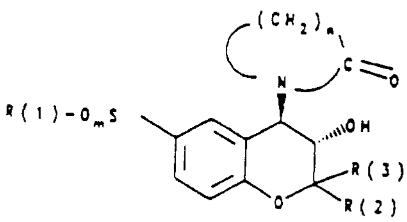

x $C_2H_5OH$

I

and pharmaceutically customary additives.

**Revendications**

**1.** Produits d'addition avec l'éthanol de formule I,

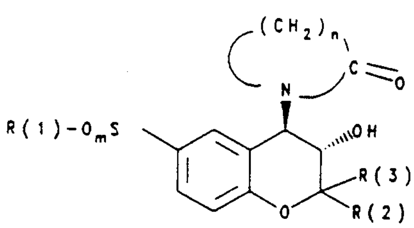

x $C_2H_5OH$

I

dans laquelle
R(1) représente un groupe phényle pouvant être substitué par 1 ou 2 groupe(s) méthyle et/ou atome(s) de chlore,

R(2) et R(3), identiques ou différents, représentent un groupe méthyle ou éthyle,
n vaut 3 ou 4, et
m vaut 1 ou 2.

**2.** Procédé de préparation d'un produit d'addition avec l'éthanol I selon la revendication 1, caractérisé en ce qu'on met un composé Ia ou un de ses solvates en contact avec de l'éthanol.

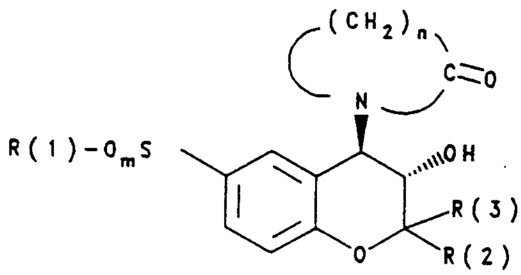

Ia

**3.** Produit d'addition avec l'éthanol de formule I selon la revendication 1,

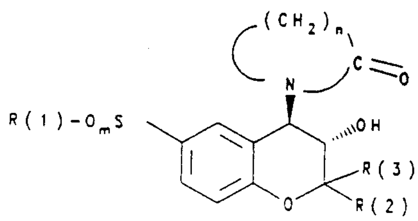

x $C_2H_5OH$

pour l'application comme produit d'inhalation dans le traitement de l'asthme.

**4.** Médicament, consistant en une quantité active d'un produit d'addition avec l'éthanol I selon la revendication 1

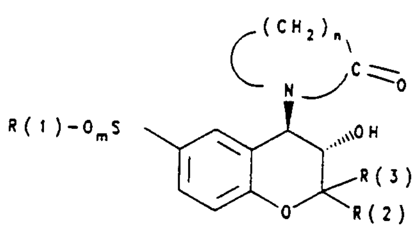

x $C_2H_5OH$

I

et des véhicules pharmaceutiques habituels.